# EUROPEAN PATENT SPECIFICATION

(11) **EP 2 796 881 B1**
(45) Date of publication and mention of the grant of the patent: **01.11.2017**
(21) Application number: 14001772.4
(22) Date of filing: 26.04.2013
(51) Int. Cl.: G01N 33/86, G01N 33/58, G01N 33/543

(54) **Platelet Allo-antigen typing and platelet antibody tests**
Plättchen-allo-antigene-Typisierung und Plättchen-Antikörpertests
Typage allo-antigène plaquettaire et tests d'anticorps plaquettaires

(43) Date of publication of application: 29.10.2014
(62) Divisional of application: 13002263.5
(73) Proprietor: Davos Diagnostics AG, 7270 Davos (CH)
(72) Inventor: Schawaller, Manfred, CH-1785 Cressier (CH); Rhyner, Claudio, CH-7270 Davos (CH); Akdis, Cezmi, CH-7265 Davos (CH); Wiki, Max, CH-1110 Morges (CH); Crameri, Reto, CH-7270 Davos (CH)
(74) Representative: Müller-Boré & Partner Patentanwälte PartG mbB

(56) References cited:
- EP-A1- 1 371 967
- EP-A1- 2 006 304
- WO-A1-01/14859
- WO-A2-2007/090630
- WO-A2-2013/013220
- US-A1- 2009 317 413

## Description

The present invention relates to methods for the detection of human antibodies against human platelet alloantigens (HPAs) on human platelets.

Blood transfusions are a routine therapy all over the world. Typically, not whole blood is transfused but only specific components of blood. Blood consists of a liquid part and a cellular part. The liquid part is called plasma and contains numerous proteins. Blood plasma is used as a therapeutic either as whole fresh or frozen plasma or as blood plasma components which are purified by biochemical means from the plasma and given as a specific therapeutic. Respective examples are immunoglobulins such as intravenous immunoglobulins (IVIG) or blood coagulation factors. The cellular fraction of blood contains three types of cells, red cells, white cells and platelets. Red cell transfusion constitutes the majority of therapeutic cellular products of blood banks followed by platelet concentrates, numbering approximately one tenth of red cell concentrates.

Blood products are from individual donors and are subject to intensive diagnostic testing. The first group of tests is to determine the surface antigens on cells, as these antigens can vary from donor to donor. These are the well known blood groups with the major antigens ABO and more than 30 other minor antigens known and described. Moreover, sera and/or plasma are tested for antibodies against these antigens. The second group of tests done routinely are tests for the absence or presence of infectious diseases in donor blood. This is to ensure that the therapeutic blood transfusion does not carry pathogenic agents. These tests are commonly done by a combination of antibody tests, such as blood plasma screening for antibodies against HIV, HBV, HCV, the ToRCH panel tests, and plasma antigen tests for pathogen specific proteins, for example an HIV p24 antigen test. Depending on the screening practice in specific countries, antigen and antibody tests can be supplemented by a nucleic acid test for viral nucleic acids based on amplification methods, for example DNA/RNA amplification using the polymerase chain reaction (PCR).

For therapeutic platelets, all the serologic and ABO blood groups tests are performed just as for red cell concentrates. However, platelets carry not only the blood group antigens, but additional extra antigens on their surface. These antigens vary from human to human and cause alloimmunizations when transfused into non-compatible human individuals. Testing for these alloantigens and for antibodies against these alloantigens in patients is not routinely done. Reasons for this are the lack of suitable diagnostic tests to determine alloantigens and suitable practical methods and tests for antibody detection.

Platelet alloantigens result from biallelic polymorphisms of membrane glycoproteins of platelets. These polymorphisms give rise to single amino acid changes in the glycoproteins having no major biological consequences. However, upon transfusion of incompatible platelets, an alloimmunization can occur and lead to clinical symptoms, described below. Another source of alloimmunization occurs during pregnancy when the pregnant woman is immunized against alloantigens from the incompatible platelets of the fetus. Allo-antibodies against platelets are one source leading to clinical symptoms, but there are also auto-antibodies directed against platelets leading to thrombocytopenia.

The platelet alloantigens are well studied proteins and most of the relevant alloantigens are described. A common nomenclature for HPAs exists describing HPA-1 to HPA-15. The convention is to designate the more common allele with "a" and the less common allele with "b". Of all the known HPAs, the HPA-1a and HPA-5b antigens are the most immunogenic alloantigens, accounting together for over 95% of clinical cases in Caucasians. In Asian populations, HPA-4 alloimmunization is a relevant problem. Other anti-platelet glycoprotein antibodies exist but are much less frequent. For a blood bank it makes sense to provide HPA-1a and HPA-5b negative platelet concentrates as they offer the opportunity for a better treatment of patients with one of the three symptoms caused by anti-platelet antibodies, *i.e.* Neonatal Allo-Immune Thrombocytopenia (NAIT), Platelet Refractoriness (PR), or Post Transfusion Purpura (PTP).

Platelet antigen typing today is done only for patients and selected platelet donors. The laboratories typically use homemade genotyping assays and phenotyping assays. The most prominent platelet alloantigens are HPA-1a and HPA-5b. A small number of HPA-1a typing tests are done by phenotyping but this approach has not found widespread use. One reason for the absence of phenotyping assays is the lack of suitable monoclonal typing reagents for determining platelet phenotypes and a lengthy and tedious procedure for phenotyping. As of today, only one monoclonal antibody for HPA-1a typing exists, however it has not been adapted widely in routine practice.

Blood bank screening of platelets has two major advantages as firstly it generates benefit by improved safety for the recipient patient and secondly it avoids inappropriate and useless donations of cost intensive platelet preparations to the wrong patient.

Platelet antibody diagnostics is performed on patient blood samples with diseases caused by platelet antibodies, such as NAIT, PR, and PTP. As the current platelet antibody tests are complicated cellular tests such as the Monoclonal Antibody Immobilization of Platelet Antigen (MAIPA) assay or Platelet Immune Fluorescence Test (PIFT), very few commercial *in vitro* diagnostic (IVD) products are available. Only specialized platelet laboratories perform these tests using homemade diagnostic tests. These specialized labs offer a service to other blood banks for platelet immunology as a regular blood bank does not commonly run these tests.

In this context, EP 2 006 304 A1 describes a monoclonal antibody selectively recognizing a human platelet alloantigen, a method for detecting at least one human platelet alloantigen using said antibody, a method for producing said antibody, and a kit and pharmaceutical composition comprising the same. Further, WO 01/14859 A1 describes a method for the determination of substances using the evanescence field method. Furthermore, US 2009/317413 A1 describes recombinant GPIIIa molecules that lack the ligand binding βA domain and bind to HPA-1 antibodies in monomeric form. Finally, WO 2013/013220 A2 describes photonic devices, systems and methods for detecting an analyte in a biological solution.

Accordingly, the technical problem underlying the present invention is to provide methods for the detection of human antibodies against platelet antigens. Said methods should be easy to perform, cost-efficient, and deliver results in a short time.

The solution to the above technical problem is achieved by the embodiments characterized in the claims.

In particular, in a first aspect, the present invention relates to a first method for the detection of human antibodies directed against human platelet alloantigens (HPAs), in a sample, comprising the steps:
(a) providing at least one surface having at least human platelets or platelet membrane fractions bound thereto, wherein the HPAs of interest that are present on said platelets or platelet membrane fractions are known;
(b) contacting said surface with said sample, wherein human antibodies directed against the HPAs that are present on said platelets or platelet membrane fractions, contained in said sample, are immobilized on said surface by specifically binding to the platelets or platelet membrane fractions of step (a);
(c) contacting said surface with a fluorescently labeled detection agent, wherein said detection agent specifically binds to human antibodies directed against the HPAs that are present on said platelets or platelet membrane fractions, and wherein said detection agent, respective human antibodies contained in said sample, and the human platelets or platelet membrane fractions of step (a) form a surface-bound complex;
(d) exciting said surface-bound complex with an evanescence field of a light source;
(e) measuring the fluorescence emitted from said surface-bound complex; and (f) detecting human antibodies against the HPAs that are present on said platelets or platelet membrane fractions based on the emitted fluorescence measured in step (e),
wherein the platelets or platelet membrane fractions are platelets or platelet fragments that comprise native glycoproteins.

In this aspect, the term "detection" is not specifically restricted and does not only include the qualitative measurement of human antibodies directed against HPAs of interest but expressly includes the quantitative determination thereof. In this context, "qualitative" as used herein means a determination of the presence or absence of an antibody of interest within a specific sample, while the term "quantitative" as used herein means a measurement of the content or amount of an antibody to be detected within a specific sample.

Antibodies to be detected with the above first method of the present invention are not particularly limited and include any antibodies directed against an HPA. Preferably, said HPA is selected from the group consisting of HPA-1a, HPA-1b, HPA-2a, HPA-2b, HPA-3a, HPA-3b, HPA-4a, HPA-4b, HPA-5a, HPA-5b, HPA-6a, HPA-6b, HPA-7a, HPA-7b, HPA-8a, HPA-8b, HPA-9a, HPA-9b, HPA-10a, HPA-10b, HPA-11a, HPA-11b, HPA-12a, HPA-12b, HPA-13a, HPA-13b, HPA-14a, HPA-14b, HPA-15a, and HPA-15b. More preferably, said HPA is selected from the group consisting of HPA-1a, HPA-1b, HPA-3a, HPA-3b, HPA-4a, HPA-4b, HPA-5a, and HPA-5b. Even more preferably, said HPA is selected from the group consisting of HPA-1a and HPA-5b, *i.e.* antibodies directed against HPA-1a and/or HPA-5b are detected.

According to the above first method of the present invention, antibodies directed against one HPA or more HPAs are detected, *e.g.* 2, 3, 4, 5, or more antibodies directed against different HPAs. In these embodiments, an according number of surfaces is provided in step (a), e.g. when antibodies against two HPAs are to be detected, two surfaces are provided. Further, all surfaces provided in step (a) are contacted in steps (b) and (c). Furthermore, all surface-bound complexes are excited in step (d), measured in step (e), and all respective antibodies are detected in step (f).

In this context, the above first method of the present invention can be performed as a multiplex method, *i.e.* more than one, e.g. 2, 3, 4, 5, 6, 7, 8, or more different platelets or platelet membrane fractions can be coated in different positions on a surface, so that e.g. all HPAs or platelet allo-antigens can be covered. For example, according to the present invention, a single microchip may be used in the above-defined first method which uses a selection of platelets or fragments thereof to cover all HPAs of interest, thus enabling the easy and quick determination of anti-HPA antibodies in a sample.

The sample to be analyzed in the above first method of the present invention is not particularly limited, provided it is a sample that contains or is suspected of containing antibodies against HPAs. Preferably, said sample is selected from the group consisting of blood plasma and blood serum.

The terms "contacting the surface" or "contacting said surface" as used in this aspect are intended to relate to the contacting of surfaces that are saturated with e.g. platelets or platelet membrane fractions, blocking agents, platelet-antibody complexes, or other complexes that can occur in the methods of the present invention. A person skilled in the art will understand that e.g. in step (b) of the above first method, the sample actually does not contact the surface, but the human platelets or platelet membrane fractions of step (a) or optionally blocking agents that are bound thereto, since said surface is saturated with said human platelets or platelet membrane fractions and optionally with blocking agents, and, therefore, is actually not accessible. However, the above terms are chosen to reflect the macroscopic viewpoint of a practitioner working the present invention, who will merely see a surface, and will know what is actually bound to said surface at any given point.

The surfaces provided in the above first method of the present invention are made of materials that are not particularly limited and are known in the art. Preferably, such materials are glass or a plastic, preferably a plastic, such as polystyrene, polypropylene, polyethylene, polyethylene terephthalate, polycycloolefin, polyacrylnitrile, polymethylmethacrylate and/or mixtures and blends of these plastics. In principle, any plastic that basically absorbs no light in the visible range is suitable. In one embodiment, the plastic can also be dyed light blue, for example, in order to filter out an emission caused by scatter light. Preferably, the above surface is the inside of a concave container, like a cuvette or a well of a microtiter plate or of a test cartridge, for example. Such cuvettes, microtiter plates and test cartridges can be obtained inexpensively by injection molding and preferably have a reaction volume of 1 to 400 µl, especially preferred 5 to 100 µl, and most preferred 10 to 40 µl. Preferably, the cuvettes, microtiter plates or test cartridges are made in one piece. In this context, the expression "test cartridge" is not specifically restricted and includes any object which may be used to provide a surface on which the human platelets or platelet membrane fractions of step (a) of the above first method of the present invention are bound and which may be contacted with the respective solutions or reactants. For example, the test cartridge may be a one-way disposable measurement cartridge that contains multiple, fully separated measurement wells, which are pre-conditioned to the specific measurement needs.

As used in this aspect, the term "bound" preferably means that the human platelets or platelet membrane fractions of step (a) are adhered to the surface by absorption (direct absorption). However, said human platelets or platelet membrane fractions may also be bound to the surface via a bridge element, for example a protein, such as an antibody, *e.g.* an antibody directed against a common platelet surface antigen such as gpVI or beta-2-microglobulin. Another possibility would be to biotinylate the platelets or platelet membrane fractions and bind them to an avidin- or streptavidin-coated surface. Said human platelets or platelet membrane fractions may also be bound to the surface by a covalent bond. This can be e.g. produced using an acrylate surface by conversion with a carbodiimide. Further, the term "bound" in the sense of the present invention includes adhesion to a surface or to another ligand, and includes both covalent and non-covalent interactions, like for example interactions based on ionic, polar or non-polar interactions.

In this aspect, the human platelets or platelet membrane fractions of step (a) can be placed on the surface by a common method. For example, said human platelets or platelet membrane fractions can be coated on the surface. After this step, the surface is preferably treated with another solution, and sites on the surface not adhered to said human platelets or platelet membrane fractions are blocked or will be blocked, for example by a protein.

According to the above first method of the present invention, the platelets or platelet membrane fractions of step (a) can form a complex, wherein this complex at least includes, besides said platelets or platelet membrane fractions, the fluorescently labeled detection agent of step (c) and the human antibody directed against an HPA to be detected. With the platelets or platelet membrane fractions of step (a) bound to the surface, said complex is "anchored" to the surface, i.e. fixed thereto and can at the same time be detected by marking it with the fluorescently labeled detection agent of step (c) containing the fluorophor.

The human platelets or platelet membrane fractions of step (a) of the above first method of the present invention are not particularly limited, provided human platelets or platelet membrane fractions are used wherein the HPAs of interest that are present on said platelets or platelet membrane fractions are known. By way of example, in case antibodies directed against HPA-1a should be detected, human platelets or platelet membrane fractions that are HPA-1a positive have to be used in step (a). Methods for obtaining human platelets or platelet membrane fractions carrying known HPAs are not particularly limited and are known in the art. One way of generating platelet membrane fractions is for example to lyse platelets, e.g. chemically and/or mechanically, and to purify membrane fractions based on their solubility in aqueous buffers. An alternative is to purify membranes based on their density, *e.g*. by sedimentation in a gravitational field by ultracentrifugation. Another method to prepare membranes is sucrose density centrifugation. In this method, solid sucrose is added to a hypotonic cell lysate to a concentration of preferably 70%. The mixture is then centrifuged, preferably in an ultracentrifuge and the membranes float on the sucrose solution. The minimal sucrose concentration to float membranes is 35%, so one can prepare step gradients by overlaying the 70% sucrose lysate with a 55% sucrose cushion and a 35% cushion on top of the 55% cushion. An alternative is to generate small membranous liposomes by sonication and/or French press optionally followed by a sizing step. In this context, the term "platelet membrane fractions" as used herein encompasses in some embodiments platelet-derived liposomes. Means for generating or obtaining platelet-derived liposomes are not particularly limited and are known in the art.

Further, the fluorescently labeled detection agent of step (c) of the above first method of the present invention is not particularly limited, provided it can specifically bind to human antibodies directed against HPAs that are present on said platelets or platelet membrane fractions.

In one particular embodiment, said detection agent is a fluorescently labeled antibody directed against human antibodies. In this embodiment, said antibody is not particularly limited, and suitable antibodies are known in the art and can be obtained *e.g*. commercially. Such antibodies are not specifically restricted concerning their isotype or their origin. Exemplary antibodies in this context could be for example murine IgG antibodies. Further, in this embodiment, the fluorescent label of said antibody is not particularly restricted, provided it can emit fluorescent light when excited with an evanescence field of a light source. Suitable fluorescent labels are known in the art. In a preferred embodiment, said fluorescent label is allo-phycocyanine (APC).

In another particular embodiment, the above detection agent is fluorescently labeled platelets or platelet membrane fractions, wherein said platelets or platelet membrane fractions carry the same HPAs as the platelets or platelet membrane fractions of step (a). Preferably, said platelets or platelet membrane fractions are identical to the platelets or platelet membrane fractions of step (a), with the exception that the former is fluorescently labeled. In this embodiment, the fluorescent label of said platelets or platelet membrane fractions is not particularly restricted, provided it can emit fluorescent light when excited with an evanescence field of a light source. Suitable fluorescent labels are known in the art. In a preferred embodiment, said fluorescent label is a lipophilic fluorescent dye that associates with the platelet membranes, *e.g*. a carbocyanine derivative. Suitable lipophilic dyes and methods of labeling platelets are not particularly limited and are known in the art. Exemplary dyes are the ebiosience CellVue dye series or the dyes available from Molecular Probes. Alternatively, platelets could be labeled via a fluorescently labeled antibody directed against a platelet surface antigen, or via biotinylation of the platelets or platelet membrane fractions and interaction with fluorescently labeled avidin or streptavidin.

In this context, it is important to note that the above first method of the present invention uses platelets or platelet fragments or platelet-derived liposomes all of which have in common the presence of native glycoproteins which are not denatured by a physical process or the activity of detergents for solubilizing. This is particularly important for the platelet glycoproteins which by nature often exist in a conformationally restricted form, and upon activation, such as by a physical treatment, a binding event or also by detergent treatment, change their conformation and are subsequently different form native glycoproteins resting on unactivated platelets in terms of their function and their characteristics as an antigen.

As used in the context of the detection agent of step (c) of the above first method of the present invention, the term "antibody" does not only refer to the respective antibody as such, but also includes any fragments and derivatives of said antibody, provided that said fragments retain the ability to specifically bind to the same epitope. Respective fragments can be Fab fragments, F(ab') fragments, or F(ab')₂ fragments. Further, instead of antibodies or antibody fragments, any other macromolecules having the ability to specifically bind to an epitope can be used. Such macromolecules include for example nucleotide aptamers, peptide aptamers, nanobodies, affibodies, anticalins, DARPins, phylomers, AdNectins, and Protein A.

The human antibodies directed against HPAs that are detected in the above first method of the present invention are not particularly limited and are preferably selected from the group consisting of IgG antibodies, IgA antibodies, IgE antibodies, and IgM antibodies. In this context, the antibodies to be detected with said method are primarily allo-antibodies. In this manner, a therapy can be directed by choosing the right platelets of step (a). However, said antibodies can also by auto-antibodies, wherein the allo-antigenic profile presented in step (a) is not relevant. Respective auto-antibodies are the cause of PTP where a preformed anti-HPA-1a antibody, typically derived from a pregnancy, undergoes evolution and becomes an auto-antibody.

In the above first method of the present invention, the steps (b) and (c) can be performed subsequently or concurrently. In particular, in a preferred embodiment, the fluorescently labeled detection agent of step (c) can be combined with the, optionally diluted, sample before the surface is contacted, so that steps (b) and (c) are performed concurrently when the respective solution is contacted with the surface. Accordingly, the surface has only to be contacted with the, optionally diluted, sample, so that steps (b) and (c) are performed concurrently.

According to a preferred embodiment, the above first method of the present invention does not require more than two, more preferably more than one, liquid manipulation steps. In this context, the expression "liquid manipulation step" as used herein generally includes any step which transfers, removes or adds a liquid from and/or to a site of action. For example, liquid manipulation steps according to the present invention include adding, *e.g*. by pipetting, a liquid into the well of a cuvette, a microtiter plate or a test cartridge, or removing a liquid from such a well. Further, according to another preferred embodiment, the above first method of the present invention does not include any washing steps.

In particular, the first method of the present invention does preferably not require any steps of removing the volume containing excess reactants, such as the fluorescently labeled detection agent of step (c), and/or washing the surface on which the platelets or platelet membrane fractions of step (a) and/or the complex to be measured are bound. This advantageously accelerates the above first method of the present invention to rapidly obtain the desired measurement results, avoids accumulation of waste liquids and operational errors when carrying out the method.

In this context, in a further embodiment of the present invention, the first method as defined above is a one-step method. According to the present invention, "one-step" method means that only one complex-forming reaction takes place, which generally requires only one or at most two liquid manipulation steps to be conducted by the operator in order to obtain the desired measurement result. Thus, further reaction steps, such as the addition of more reagents after initial complex-formation are advantageously avoided.

Moreover, a further embodiment of the present invention relates to a method as defined above, wherein said method does not include liquid agitation. In this context, the expression "liquid agitation" is not specifically limited and includes any type of agitation which occurs in the liquid, except for natural diffusion. For example, liquid agitation in the sense of the present invention includes shaking, pumping, stirring, ultrasonic agitation, etc. By avoiding any liquid agitation, except for natural occurring diffusion, the method can be carried out in a simple and effective manner, and thus avoids complicated technical setups including respective agitation devices.

According to a further embodiment of the above-defined first method, the result determined in step (f) is obtained within 30 minutes, preferably 20 minutes, more preferably 10 minutes or less starting from the beginning of step (b). According to the present invention, the qualitative or quantitative result is obtained within 30 minutes, preferably 20 minutes, more preferably 10 minutes or less after the solution containing the sample and the fluorescently labeled detection agent of step (c) has been contacted with the surface to which the platelets or platelet membrane fractions of step (a) are bound. Preferably, the result is obtained within 9 minutes or less, 8 minutes or less or 7 minutes or less. Further examples include periods of 6 minutes or less, 5 minutes or less or 4 minutes or less.

According to a further embodiment of the above first method of the present invention, said surface is contacted prior to step (d) with at least one dye that absorbs in the absorption and/or emission range of the fluorescent label of the detection agent of step (c). According to the present invention, excitation of the fluorophor in the volume, *i.e*. of fluorophor not part of the surface-bound complex, can be suppressed if the solution to be placed in contact with the surface has at least one dye added to it that has an absorption in the absorption and/or emissions range of said fluorophor. The absorption of the dye added to the volume is coordinated with the absorption and/or emission range of said fluorophor. One individual dye or a mixture of dyes can be used. The absorption range of the fluorophor generally correlates with the wavelength of the light source used. It is not necessary that the dye has an absorption maximum in this special range; a shoulder in the absorption spectrum can suffice. For example, if fluorophors like APC or Cy5 are used, the dye used can have absorption between 600 nm and 700 nm, like for example Brilliant Blue FCF. Another example of a dye that can be used in this respect is Brilliant Black BN. The concentration of dye added depends on both the absorption coefficient of each dye in solution and the frequency of the light radiated. The concentration of dye can be adjusted, depending on the dye, so that the penetrating light can basically be absorbed to an extent of over 90% absorbance within 0.1 mm above the surface.

According to steps (d) to (f) of the above first method of the present invention, the surface-bound complex generated in step (a) to (c) of said method is excited with an evanescence field of a light source, the fluorescence emitted from said complex is measured, and the human antibodies against the HPAs of interest are detected based on the measured emitted fluorescence, wherein a positive fluorescence signal indicates the presence of said complex and, thus, the presence of human antibodies directed against the HPAs of interest.

In this context, the term "light source" as used herein is not specifically restricted and includes any light source which is suited to create an evanescence field and excite the fluorophor bound to the detection agent of step (c). For example, monochromatic light may be used as the light source. Light should be used that has a wavelength that preferably does not interfere with the emission of the fluorophor and preferably intersects with the absorption band of the dye. A laser is especially preferred as a light source, whose light emits a wavelength of 635 nm.

The above first method of the present invention is based on a new technology for analysis of biomolecules using the evanescence field excitation of bound fluorophors and measuring directly the binding events in real time as described in the European patents EP 1 079 226 B1, EP 1 204 856 B1, and EP 1 371 967 B1. The functional principle of this evanescence field biosensor is the interaction of a surface-bound analyte with a ligand in solution by a non-covalent interaction. The ligand in solution is attached covalently to a fluorescent molecule. Preferred excitation of the fluorescent molecule is with coherent light such as generated by *e.g*. a laser diode and then the emitted photons are detected. Suitable fluorophors for this are for example Cy5, Alexa dyes or the light-harvesting protein Allo-phycocyanine (APC).

The biochemical reaction takes place in a small well, similar to an ELISA well in form and dimensions with an additional optical entity on the bottom. As in ELISA, the biosensor well and its optical bottom part are produced by injection molding of thermoplastic material, like *e.g*. polystyrene. Major differences between the ELISA method and the evanescence biosensor methods are the labels of the ligand in solution. For ELISA, the label is an enzyme and for the evanescence technology the ligand is labeled with a fluorescent molecule. Measurement of the bound fluorophor, *i.e*. the fluorescing ligand in solution binding to the analyte immobilized on the evanescent surface, is achieved by specific excitation of the bound fluorescently labeled ligand using the evanescence field excitation principle. Selective excitation of the bound fluorophor occurs by an evanescence field of light using an optical construction resulting in a specific excitation of an approximately 200 nm thick layer of liquid located at the bottom of the well and not exciting the fluorescently labeled ligand in excess present in the solution above the binding surface. Only fluorescently labeled ligands residing in the evanescent field are excited and emit photons. This method allows the observation of a binding reaction of the soluble ligand with the immobilized ligand in real time.

While most immune assays used in *in vitro* diagnostic testing have a long and tedious procedure involving incubation times, washes, addition of secondary or tertiary reagents or solutions to obtain a result for a biomarker, the evanescence biosensor allows to perform *in vitro* diagnostic assays in short time. The complex and costly instrumentation of ELISA automates or for bead based immune assays are neither required nor necessary when using the evanescence biosensor for diagnostic applications.

The first method of the present invention can be practiced in connection with a first diagnostic test device for carrying out said method, comprising at least one surface having at least the human platelets or platelet membrane fractions of step (a) bound thereto, wherein the HPAs that are present on said platelets or platelet membrane fractions are known.

In this context, the surface, the human platelets or platelet membrane fractions of step (a), and the fluorescently labeled detection agent of step (c) are as defined above. Further, the definitions given above apply also to this device.

Surprisingly and unexpectedly, when considering the geometrical dimensions of an evanescence field and the dimensions of typical platelets, the evanescence field being 200 nm in height and the platelets used for coating the sensing surface having a diameter of 1 to 4 micrometers, one still can observe a signal. Just a minority of the coated platelet and the platelet membrane is within the evanescent field and, therefore, can absorb and consequently emit fluorescent signals, whereas the majority of the 2-3 micrometer platelet and the platelet surface antigens are outside of the evanescent field. This indicates a rather high stability of the system towards the dimensions of the biological analyte. Furthermore, it should again be noted that the coated platelets and platelet fragments are rich in enzymes and enzymatic activities such as peroxidases and phosphatase, which are enzymes commonly used as labels for ELISA tests. However, the fluorescence detection using evanescence excitation avoids any problems that could result from those contaminating enzymes altogether.

Preferably, the above first diagnostic test device has the form of a cuvette, microtiter plate or test cartridge, preferably being made of glass or a plastic, preferably a plastic, such as polystyrene, polypropylene, polyethylene, polyethylene terephthalate, polycycloolefin, polyacrylnitrile, polymethylmethacrylate and/or mixtures and blends of these plastics. In principle, any plastic that basically absorbs no light in the visible range is suitable. The plastic can also be dyed light blue, for example, in order to filter out an emission caused by scatter light. Plastic cuvettes, microtiter plates and test cartridges can be obtained inexpensively by injection molding and preferably have a reaction volume of 1 to 400 µl, especially preferred 5 to 100 µl, and most preferred 10 to 40 µl. Preferably, the cuvettes, microtiter plates or test cartridges are made in one piece.

In this context, the expression "test cartridge" is not specifically restricted and includes any object which may be used to provide a surface on which the human platelets or platelet membrane fractions of step (a) are bound and which may be contacted with the respective solutions or reactants. For example, the test cartridge may be a one-way disposable measurement cartridge that contains multiple, fully separated measurement wells, which are pre-conditioned to the specific measurement needs.

In this context, as the typical test cartridge has multiple wells, multiple assays can be run in parallel.

In a second aspect, the present invention relates to a second method for the concurrent detection of (i) at least one human platelet alloantigen (HPA) on human platelets contained in a sample, and (ii) human antibodies directed against human platelet alloantigens (HPAs), in a sample, comprising the steps of:
(a) performing a method for the detection of at least one human platelet alloantigen (HPA) on human platelets contained in a sample as defined hereinafter; and
(b) performing the above first method of the present invention.

In this aspect, all preferred embodiments and definitions as given above or below for said methods apply.

In particular, the method for the detection of at least one HPA on human platelets contained in a sample which is part of the second method of the present invention is a method for the detection of at least one HPA on human platelets contained in a sample which is anti-coagulated whole blood in a concentration of more than 25% comprising the steps:
(a) providing at least one surface having at least an antibody bound thereto, wherein said antibody is directed against the glycoprotein carrying the at least one HPA to be detected;
(b) lysing the platelets contained in the sample and solubilizing the membrane proteins of said platelets;
(c) contacting said surface with the lysed and solubilized sample of step (b), wherein respective glycoproteins contained in said sample are immobilized on said surface by specifically binding to the antibody of step (a);
(d) contacting said surface with a fluorescently labeled antibody, wherein said antibody is directed against the at least one HPA to be detected, and wherein said antibody, respective glycoproteins contained in said sample, and the antibody of step (a) form a surface-bound complex;
(e) exciting said surface-bound complex with an evanescence field of a light source;
(f) measuring the fluorescence emitted from said surface-bound complex; and (g) detecting the at least one HPA based on the emitted fluorescence measured in step (f).

In this aspect, the term "detection" is not specifically restricted and does not only include the qualitative measurement of HPAs of interest but expressly includes the quantitative determination thereof. In this context, "qualitative" as used herein means a determination of the presence or absence of an HPA of interest within a specific sample, while the term "quantitative" as used herein means a measurement of the content or amount of an HPA to be detected within a specific sample.

HPAs to be detected with the above method are not particularly limited and are known in the art. Preferably, the at least one HPA is selected from the group consisting of HPA-1a, HPA-1b, HPA-2a, HPA-2b, HPA-3a, HPA-3b, HPA-4a, HPA-4b, HPA-5a, HPA-5b, HPA-6a, HPA-6b, HPA-7a, HPA-7b, HPA-8a, HPA-8b, HPA-9a, HPA-9b, HPA-10a, HPA-10b, HPA-11a, HPA-11b, HPA-12a, HPA-12b, HPA-13a, HPA-13b, HPA-14a, HPA-14b, HPA-15a, and HPA-15b. More preferably, the at least one HPA is selected from the group consisting of HPA-1a, HPA-1b, HPA-3a, HPA-3b, HPA-4a, HPA-4b, HPA-5a, and HPA-5b. Even more preferably, the at least one HPA is selected from the group consisting of HPA-1a and HPA-5b, *i.e*. HPA-1a and/or HPA-5b are detected.

According to the above method, at least one HPA is detected. Thus, also more than one HPA can be detected, *e.g*. 2, 3, 4, 5, or more HPAs. In these embodiments, an according number of surfaces is provided in step (a), *e.g*. when two HPAs are to be detected, two surfaces are provided. Further, all surfaces provided in step (a) are contacted in steps (c) and (d), and the respective fluorescently labeled antibodies directed against the HPAs to be detected are used in step (d). Furthermore, all surface-bound complexes are excited in step (e), measured in step (f), and all respective HPAs are detected in step (g).

The sample to be analyzed in the above method is anti-coagulated whole blood.

According to the above-defined method, anti-coagulated whole blood is used in the final test in concentrations of more than 25%, e.g. more than 33%, more than 50% or even more than 80%.

However, surprisingly, it is possible to use an anti-coagulated whole blood sample in the above method with obtaining reliable and excellent test results. The whole blood sample is used above a concentration of 25 % in the final tests, wherein it is obvious that this may give rise to non-specific signals due to unwanted biochemical interactions. However, unexpectedly, very low biochemical background has been observed, still allowing to detect specifically and selectively the platelet glycoprotein. Even more important to note is that the above method allows to detect and distinguish with high sensitivity glycoproteins differing from each other by only one single amino acid polymorphism. Furthermore, the ability of the tests to give specific signal even in the presence of a large excess of potential fluorophors in a lysed whole blood solution, namely hemoglobin, cytoplasmic proteins from erythrocytes and other blood cells as well the large amount of human plasma proteins, is surprising. An autofluorescence signal resulting from any component of the whole blood sample is not observed. Remarkable is also that biological degradation processes occurring in whole blood when stored at 4°C do not have a significant influence on the assay signal. Blood sample stored at 4°C for over one year can be used in the assay and give a correct result. This indicates again the lack of autofluorescence phenomena and the stability of the platelet glycoproteins upon storage and aging.

The terms "contacting the surface" or "contacting said surface" as used in this aspect are intended to relate to the contacting of surfaces that are saturated with e.g. antibodies, blocking agents, antibody-antigen complexes, or other complexes that can occur in the methods of the present invention. A person skilled in the art will understand that *e.g*. in step (c) of the above method, the sample actually does not contact the surface, but the antibodies of step (a) or optionally blocking agents that are bound thereto, since said surface is saturated with said antibodies and optionally with blocking agents, and, therefore, is actually not accessible. However, the above terms are chosen to reflect the macroscopic viewpoint of a practitioner working the present invention, who will merely see a surface, and will know what is actually bound to said surface at any given point.

The surfaces provided in the above method are made of materials that are not particularly limited and are known in the art. Preferably, such materials are glass or a plastic, preferably a plastic, such as polystyrene, polypropylene, polyethylene, polyethylene terephthalate, polycycloolefin, polyacrylnitrile, polymethylmethacrylate and/or mixtures and blends of these plastics. In principle, any plastic that basically absorbs no light in the visible range is suitable. In one embodiment, the plastic can also be dyed light blue, for example, in order to filter out an emission caused by scatter light. Preferably, the above surface is the inside of a concave container, like a cuvette or a well of a microtiter plate or of a test cartridge, for example. Such cuvettes, microtiter plates and test cartridges can be obtained inexpensively by injection molding and preferably have a reaction volume for the liquid of 1 to 400 µl, especially preferred 5 to 100 µl, and most preferred 10 to 40 µl. Preferably, the cuvettes, microtiter plates or test cartridges are made in one piece. In this context, the expression "test cartridge" is not specifically restricted and includes any object which may be used to provide a surface on which the antibody of step (a) of the above method is bound and which may be contacted with the respective solutions or reactants. For example, the test cartridge may be a one-way disposable measurement cartridge that contains multiple, fully separated measurement wells, which are pre-conditioned to the specific measurement needs.

As used in this aspect, the term "bound" preferably means that the antibody of step (a) is adhered to the surface by absorption (direct absorption). However, said antibody may also be bound to the surface via a bridge element, for example a protein, such as a further antibody or an antigen. Said antibody may also be bound to the surface by a covalent bond. This can be *e.g*. produced using an acrylate surface by conversion with a carbodiimide. Further, the term "bound" in the sense of the present invention includes adhesion to a surface or to another ligand, and includes both covalent and non-covalent interactions, like for example interactions based on ionic, polar or non-polar interactions.

In this aspect, the antibody of step (a) can be placed on the surface by a common method. For example, said antibody can be coated on the surface. After this step, the surface is preferably treated with another solution, and sites on the surface not adhered to said antibody are blocked or will be blocked, for example by another protein or a chemical compound such as a detergent.

According to the above method, the antibody of step (a) can form a complex, wherein this complex at least includes, besides said antibody, the fluorescently labeled antibody of step (d) and the glycoprotein carrying the HPA to be detected. With the antibody of step (a) bound to the surface, the complex with the glycoprotein carrying the HPA to be detected is "anchored" to the surface, *i.e.* fixed thereto and can at the same time be detected by marking it with the antibody of step (d) containing the fluorophor.

The antibody of step (a) of the above method is not particularly limited, provided it is directed against the glycoprotein carrying the at least one HPA to be detected, *i.e.* provided it specifically binds to said glycoprotein. Suitable antibodies are known in the art and can be obtained *e.g*. commercially. Such antibodies are not specifically restricted concerning their isotype or their origin. Exemplary antibodies in this context could be for example murine IgG antibodies. Glycoproteins of interest in this respect are known in the art and include in particular gpIIbIIIa, gpIaIIa, gpIbIX and HLA/beta-2-microglobulin. Particular antibodies that can be used in this respect are the monoclonal anti-gpllbllla antibody RFGP56 and the monoclonal anti-gpIaIIa antibody AK7. Other suitable antibodies are for example the anti-gpllbllla clone P2 Immuntech or the anti-gplalla clones Gi9 and Y418. Many more suitable antibodies can be found by the person skilled in the art.

Further, the fluorescently labeled antibody of step (d) of the above method is not particularly limited, provided it is directed against the HPA to be detected, *i.e*. provided it specifically binds to said HPA. Suitable antibodies are not specifically restricted concerning their isotype and origin, and are known in the art. Further, the fluorescent label of said antibody is not particularly restricted, provided it can emit fluorescent light when excited with an evanescence field of a light source. Suitable fluorescent labels are known in the art. In a preferred embodiment, said fluorescent label is allo-phycocyanine (APC).

Preferably, in the above method, the antibody of step (a) and the antibody of step (d) have different specificities, *i.e.* they bind to different epitopes.

In the above method, the antibody of step (a) and the antibody of step (d) can be exchanged, *i.e.* the antibody of step (a) is directed against the at least one HPA to be detected, and the fluorescently labeled antibody of step (d) is directed against the glycoprotein carrying the at least one HPA to be detected. In this particular embodiment, said antibodies, as well as the fluorescent label, are as defined above.

As used herein, the term "antibody" does not only refer to the respective antibody as such, but also includes any fragments and derivatives of said antibody, provided that said fragments retain the ability to specifically bind to the same epitope. Respective fragments can be Fab fragments, F(ab') fragments, or F(ab')₂ fragments. Further, instead of antibodies or antibody fragments, any other macromolecules having the ability to specifically bind to an epitope can be used. Such macromolecules include for example nucleotide aptamers, peptide aptamers, nanobodies, affibodies, anticalins, DARPins, phylomers, AdNectins, and Protein A.

According to the above method, in step (b) platelets contained in the sample are lysed and the membrane proteins of said platelets solubilized. Methods and agents for lysing and solubilization are not particularly limited and are known in the art. They include for example the treatment of said platelets with suitable concentrations of surfactants such as Tween 20 (polysorbate 20; polyoxyethylene (20) sorbitan monolaurate) or NP40 (nonyl phenoxypolyethoxylethanol).

In the above method, the steps (c) and (d) can be performed subsequently or concurrently. In particular, in a preferred embodiment, the fluorescently labeled antibody of step (d) can be contained in the lysing and solubilization solution formed in step (b), so that steps (c) and (d) are performed concurrently when the respective solution is contacted with the surface.

Preferably, the above method does not require more than two, more preferably more than one, liquid manipulation steps. In this context, the expression "liquid manipulation step" as used herein generally includes any step which transfers, removes or adds a liquid from and/or to a site of action. For example, liquid manipulation steps according to the present invention include adding, *e.g*. by pipetting, a liquid into the well of a cuvette, a microtiter plate or a test cartridge, or removing a liquid from such a well. Further, according to another preferred embodiment, the above method does not include any washing steps.

In particular, the above method does preferably not require any steps of removing the volume containing excess reactants, such as the fluorescently labeled antibody of step (d), and/or washing the surface on which the antibody of step (a) and/or the complex to be measured are bound. This advantageously accelerates the above method to rapidly obtain the desired measurement results, avoids accumulation of waste liquids and operational errors when carrying out the method.

In this context, the method as defined above is preferably a one-step method. According to the present invention, "one-step" method means that only one complex-forming reaction takes place, which generally requires only one or at most two liquid manipulation steps to be conducted by the operator in order to obtain the desired measurement result. Thus, further reaction steps, such as the addition of more reagents after initial complex-formation are advantageously avoided.

Moreover, in a further embodiment of the method as defined above, said method does not include liquid agitation. In this context, the expression "liquid agitation" is not specifically limited and includes any type of agitation which occurs in the liquid, except for natural diffusion. For example, liquid agitation in the sense of the present invention includes shaking, pumping, stirring, ultrasonic agitation, etc. By avoiding any liquid agitation, except for natural occurring diffusion, the method can be carried out in a simple and effective manner, and thus avoids complicated technical setups including respective agitation devices.

According to a further embodiment of the above-defined method, the result determined in step (g) is obtained within 30 minutes, preferably 20 minutes, more preferably 10 minutes or less starting from the beginning of step (c). According to the present invention, the qualitative or quantitative result is obtained within 30 minutes, preferably 20 minutes, more preferably 10 minutes or less after the solution containing the lysed and solubilized sample and the fluorescently labeled antibody of step (d) has been contacted with the surface to which the antibody of step (a) is bound. Preferably, the result is obtained within 9 minutes or less, 8 minutes or less or 7 minutes or less. Further examples include periods of 6 minutes or less, 5 minutes or less or 4 minutes or less.

According to a further embodiment of the above method, said surface is contacted prior to step (e) with at least one dye that absorbs in the absorption and/or emission range of the fluorescent label of the antibody of step (d). According to the present invention, excitation of the fluorophor in the volume, *i.e.* of fluorophor not part of the surface-bound complex, can be suppressed if the solution to be placed in contact with the surface has at least one dye added to it that has an absorption in the absorption and/or emissions range of said fluorophor. The absorption of the dye added to the volume is coordinated with the absorption and/or emission range of said fluorophor. One individual dye or a mixture of dyes can be used. The absorption range of the fluorophor generally correlates with the wavelength of the light source used. It is not necessary that the dye has an absorption maximum in this special range; a shoulder in the absorption spectrum can suffice. For example, if fluorophors like APC or Cy5 are used, the dye used can have absorption between 600 nm and 700 nm, like for example Brilliant Blue FCF. Another example of a dye that can be used in this respect is Brilliant Black BN. The concentration of dye added depends on both the absorption coefficient of each dye in solution and the frequency of the light radiated. The concentration of dye can be adjusted, depending on the dye, so that the penetrating light can basically be absorbed to an extent of over 90% absorbance within 0.1 mm above the surface.

According to steps (e) to (g) of the above method, the surface-bound complex generated in step (a) to (d) of said method is excited with an evanescence field of a light source, the fluorescence emitted from said complex is measured, and the at least one HPA of interest is detected based on the measured emitted fluorescence, wherein a positive fluorescence signal indicates the presence of said complex and, thus, the presence of the at least one HPA of interest.

In this context, the term "light source" as used herein is not specifically restricted and includes any light source which is suited to create an evanescence field and excite the fluorophor bound to the antibody of step (d). For example, monochromatic light may be used as the light source. Light should be used that has a wavelength that preferably does not interfere with the emission of the fluorophor and preferably intersects with the absorption band of the dye. A laser is especially preferred as a light source, whose light emits a wavelength of 635 nm.

The above method is based on a new technology for analysis of biomolecules using the evanescence field excitation of bound fluorophors and measuring directly the binding events in real time as described in the European patents EP 1 079 226 B1, EP 1 204 856 B1, and EP 1 371 967 B1. The functional principle of this evanescence field biosensor is the interaction of a surface-bound analyte with a ligand in solution by a non-covalent interaction. The ligand in solution is attached covalently to a fluorescent molecule. Preferred excitation of the fluorescent molecule is with coherent light such as generated by *e.g.* a laser diode and then the emitted photons are detected. Suitable fluorophors for this are for example Cy5, Alexa dyes or the light-harvesting protein Allo-phycocyanine (APC).

The biochemical reaction takes place in a small well, similar to an ELISA well in form and dimensions with an additional optical entity on the bottom. As in ELISA, the biosensor well and its optical bottom part are produced by injection molding of thermoplastic material, like *e.g*. polystyrene. Major differences between the ELISA method and the evanescence biosensor methods are the labels of the ligand in solution. For ELISA, the label is an enzyme and for the evanescence technology the ligand is labeled with a fluorescent molecule. Measurement of the bound fluorophor, *i.e.* the fluorescing ligand in solution binding to the analyte immobilized on the evanescent surface, is achieved by specific excitation of the bound fluorescently labeled ligand using the evanescence field excitation principle. Selective excitation of the bound fluorophor occurs by an evanescence field of light using an optical construction resulting in a specific excitation of an approximately 200 nm thick layer of liquid located at the bottom of the well and not exciting the fluorescently labeled ligand in excess present in the solution above the binding surface. Only fluorescently labeled ligands residing in the evanescent field are excited and emit photons. This method allows the observation of a binding reaction of the soluble ligand with the immobilized ligand in real time.

While most immune assays used in *in vitro* diagnostic testing have a long and tedious procedure involving incubation times, washes, addition of secondary or tertiary reagents or solutions to obtain a result for a biomarker, the evanescence biosensor allows to perform *in vitro* diagnostic assays in short time. The complex and costly instrumentation of ELISA automates or for bead based immune assays are neither required nor necessary when using the evanescence biosensor for diagnostic applications.

The above method can be practiced in connection with a diagnostic test device for carrying out said method, comprising at least one surface having at least the antibody of step (a) bound thereto.

In this context, the surface, the antibody of step (a), suitable lysing and solubilizing agents used in step (b), and the fluorescently labeled antibody of step (d) are as defined above. Further, the definitions given above apply also to this device.

Preferably, the above diagnostic test device has the form of a cuvette, microtiter plate or test cartridge, preferably being made of glass or a plastic, preferably a plastic, such as polystyrene, polypropylene, polyethylene, polyethylene terephthalate, polycycloolefin, polyacrylnitrile, polymethylmethacrylate and/or mixtures and blends of these plastics. In principle, any plastic that basically absorbs no light in the visible range is suitable. The plastic can also be dyed light blue, for example, in order to filter out an emission caused by scatter light. Plastic cuvettes, microtiter plates and test cartridges can be obtained inexpensively by injection molding and preferably have a reaction volume of 1 to 400 µl, especially preferred 5 to 100 µl, and most preferred 10 to 40 µl. Preferably, the cuvettes, microtiter plates or test cartridges are made in one piece.

In this context, the expression "test cartridge" is not specifically restricted and includes any object which may be used to provide a surface on which the antibody of step (a) is bound and which may be contacted with the respective solutions or reactants. For example, the test cartridge may be a one-way disposable measurement cartridge that contains multiple, fully separated measurement wells, which are pre-conditioned to the specific measurement needs.

In this context, as the typical test cartridge has multiple wells, multiple assays can be run in parallel. Respective examples include the typing of several allo-antigens at the same time, optionally including positive and negative internal controls for controlling the performance of the tests. Another example could be the typing of one or more allo-antigens and the establishment of a calibration curve at the same time.

In the present application, methods and tests for diagnostic platelet antigen typing for antibody assays have been described based on the evanescence biosensor technology. This invention offers platelet phenotyping assays and antibody assays to be performed with just one or two simple manipulations and giving a result in ten minutes or less. The present invention does not need sophisticated instrumentation or in depth knowledge of platelet immunology on the side of the practitioner. The assay formats are unique products and give the end user a result in ten minutes or less. To perform the assay according to some embodiments, a user must only add a sample and measure in the biosensor reader. This will be the only manual working step required and a result is available without any further manipulations such as washing and pipetting.

The figures show:

### Figure 1:

### Test formats for antigen typing assays and antibody detection assays

(A) Assay format for the antigen typing assays. An anti-glycoprotein antibody (a) is immobilized on the sensor surface at the bottom of the well. The platelet membrane glycoprotein (b) from the sample is bound in a one-step assay with the glycoprotein allele specific reagent (c) covalently modified with a fluorophor (asterisk).
(B) Assay format for one-step and two-step antibody assays. Platelets (a) carrying the glycoprotein (d) are immobilized on the sensor surface and reacted with antibodies (b) in the patient sample. These are detected with a fluorescent anti-immunoglobulin antibody (c).
(C) Assay format for alternative one-step and two-step antibody assays. Platelets (a) carrying the glycoprotein (d) are immobilized on the sensor surface and reacted with antibodies (b) in the patient sample. These are detected with fluorescently labeled platelets (c) carrying the same glycoprotein (d).

### Figure 2:

### Assay scheme for a MAIPA-based assay

A MAIPA assay has a cellular assay part binding the platelets with anti-glycoprotein antibodies and IgG from the human plasma (A) and a detection part (B).
(A) A platelet (a) with the platelet glycoprotein (b) is brought into contact with the human plasma sample to be analyzed (e). Binding of the human anti-platelet antibody (d) occurs, leaving behind the serum with a reduced or depleted anti-platelet antibody (f). Excess human plasma is washed off manually. Subsequently, a monoclonal antibody (c) directed against the platelet glycoprotein under examination is added which binds said glycoprotein on the platelet surface. Typically four reactions for four different glycoproteins are done with monoclonal antibodies against gpllbllla, gplalla, gpIbIX and HLA/beta-2-microglobulin. After the complex is formed, (b) and (c) interact and excess unbound monoclonal antibody (c) is washed off manually. Using a detergent containing lysis buffer, the tri-molecular complex made of glycoprotein specific antibody (c), platelet glycoprotein (b) and human immunoglobulin (d) is solubilized.
(B) Detection of the tri-molecular complex (b), (c), (d) in a one-step evanescence assay. An anti-mouse IgG antibody (g) is immobilized on the bottom of the biosensor well and incubated with the tri-molecular analyte complex (b), (c), (d) and detected with the anti-human immunoglobulin fluorophor conjugate (h).

### Figure 3:

### Results obtained with an HPA-1a typing assay

HPA-1a positive and HPA-1a negative blood samples were tested in both a commercial HPA-1a typing assay (DiaMed, Cressier FR, Switzerland), (OD 630nm, x-axis), and the evanescence biosensor assay according to the present invention (Fluorescence dC cps, y-axis).

### Figure 4:

### Results obtained with an HPA-5b typing assay

Known HPA-5b positive and HPA-5b negative blood samples were tested with the evanescence biosensor assay according to the present invention (Fluorescence dC cps, y-axis).

### Figure 5:

### ELISA-based MAIPA assay and MAIPA assay of the present invention

This figure shows the comparison of MAIPA assays with either ELISA detection signal (Optical Density OD 630nm on the x-axis) and the evanescence biosensor result according to the present invention (Fluorescence dc cps on the y-axis).

### Figure 6:

### ELISA-based MAIPA assay and MAIPA assay of the present invention

This figure shows the comparison of MAIPA assays where four glycoproteins are tested in individual tubes and an evanescence biosensor MAIPA according to the present invention. For the evanescence biosensor tests four different monoclonal antibodies reacting with the four individual platelet glycoproteins gpIIbIIIa, gpIaIIa, gpIbIX and HLA were combined in one tube binding to a pool of platelets representing all possible alloantigens. Detection is done as previously described with the evanescence biosensor. ELISA detection signal (Optical Density OD 630nm on the x-axis) and the biosensor result (Fluorescence dc cps on the y-axis).

### Figure 7:

### Two-step antibody assay

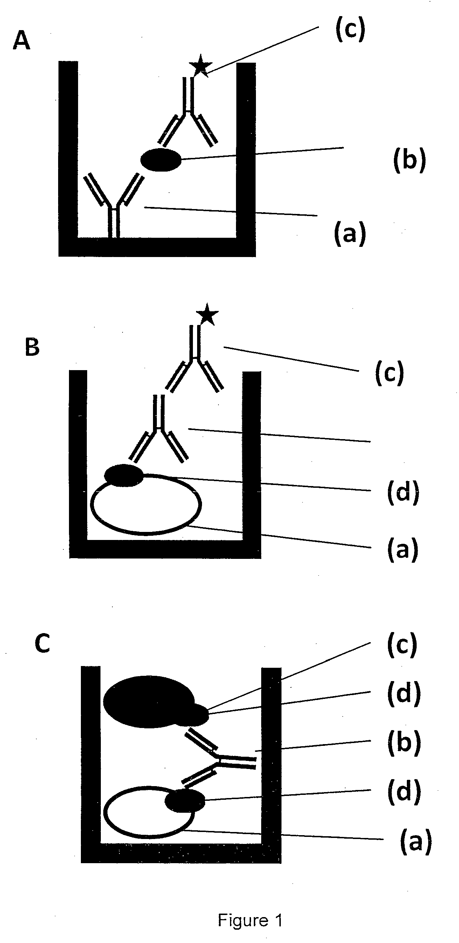

Results of a two-step antibody assay, done according to the scheme shown in Figure 1B. Platelets with either phenotype HPA-1a positive or HPA-1a negative (two samples each) were coated on the biosensor surface and reacted with diluted anti-human HPA-1a monoclonal antibody in various concentrations. Wells are washed and bound humanized anti-HPA-1a antibody is detected with a secondary anti-human IgG fluorophor conjugate. Anti HPA-1a concentrations on the x-axis, the observed signal as Fluorescence dC cps is shown on the y-axis.

### Figure 8:

### Two-step antibody assay

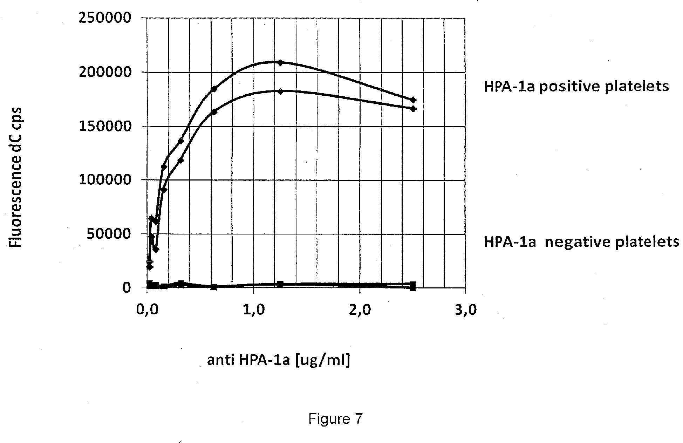

This figure shows the result of a two-step antibody assay according to the scheme shown in Figure 1B and essentially identical to the example shown in Figure 7. Six human plasma samples were tested against HPA-1a positive and against HPA-1a negative platelets coated in the evanescent biosensor. The x-axis shows the individual plasma and the obtained signal is plotted on the y-axis.

### Figure 9:

### One-step antibody assay

This figure shows an identical assay format wherein the washing step was omitted and the two-step-assay shown in Figure 7 is done as a one-step assay. Three coatings were made, HPA1a+5b+ platelets, HPA1a-5b- platelets and a non-coated control designated 0-. The obtained signal is shown on the y-axis as fluorescence dc cps.

### Figure 10:

### Double antigen antibody sandwich assay for Toxoplasmose antibodies

This test is performed by coating Toxoplasmose antigen on the biosensor surface and another aliquot of Toxoplasmose antigen is labeled with APC in solution. The IgG is biofunctional and one arm reacts with bound Toxoplasmose antigen and the other arm of an individual IgG reacts with the APC-labeled antigen. As such, as cartridge is formed giving rise to a signal. Surprisingly, this works as a one-step assay. Shown is a titration of Toxoplasmose antibody standard on the x-axis and the 10 min fluorescence signal on the y-axis. Sensitivity is found down to about 5 IU as required.

The present invention will be further illustrated by the following examples without being limited thereto.

### Examples

### Example 1 (Reference Example):

### HPA-1a typing assay

An HPA-1a evanescence biosensor test is performed by reacting an anti-gpIIbIIIa antibody coated surface (anti-gpllbllla monoclonal antibody RFGP56), one part of EDTA anti-coagulated blood and two parts of a detection mixture containing the anti HPA-1a specific antibody conjugated to Allo-phycocyanin (APC).

An evanescence biosensor chip is coated with a solution of RFGP56 in PBS. Coating is done by diluting the RFGP56 stock solution to a 10 microgram per milliliter solution in PBS, adding 30 microliters of this solution to each well and incubating for 2 hours at room temperature. The coating solution is then removed, the well is washed 3 times with PBS and finally 50 microliters of blocking solution is added to the well. The blocking solution is a 1 % solution of BSA in PBS and contains 0.25 % Tween20. Blocking is for approximately 1 hour at room temperature and is terminated by removing the blocking solution and adding the sample solution to be measured. One part of EDTA blood was mixed with two parts of a detection buffer containing anti-HPA-1a APC at 7.5 micrograms per milliliter in a PBS buffer supplemented with 4.5% BSA, 3% Sucrose, 15mM EDTA, 0.08% Tween20, 0.9% NP40, 0.75% PEG6000, 1.5% PVPK90, 0.2mg/ml IIR and 0.06% Brilliant Black BN. 25 microliters of the mixture was put in a coated well and fluorescence was measured in real-time over 10 minutes using evanescence biosensor instrument and device described in EP1079226, EP1204856, EP1371967.

The increase in measured photons is given as a whole number and is the increase of counted photons within the first 10 minutes of the reaction for one specific well. The increase of photons measured in one well is given as the change of photons from time zero seconds to time 600 seconds as 'delta counts' (dc) with the measuring unit counts per second or abbreviated 'cps'. The photons emitting from one specific well are measured during the biochemical reaction at time intervals, each measurement is for one second, but any value between 10 milliseconds or 20 seconds per measurement point can also be used. The measured photons are plotted in a graph on the y-axis against the time on the x-axis and this procedure is performed for each well individually. Then a linear regression curve is generated by the software of the reader instrument and the average increase of photons per 10 minute observation time is calculated automatically.

A dc of 23099 counts per second, cps, the measured photons in one second, means for this specific well the number of photons measured has increased by 23099 cps within the 10 minutes of measurement. The increase in photon over time, dc in cps, is now a measure for the amount of analyte in the sample.

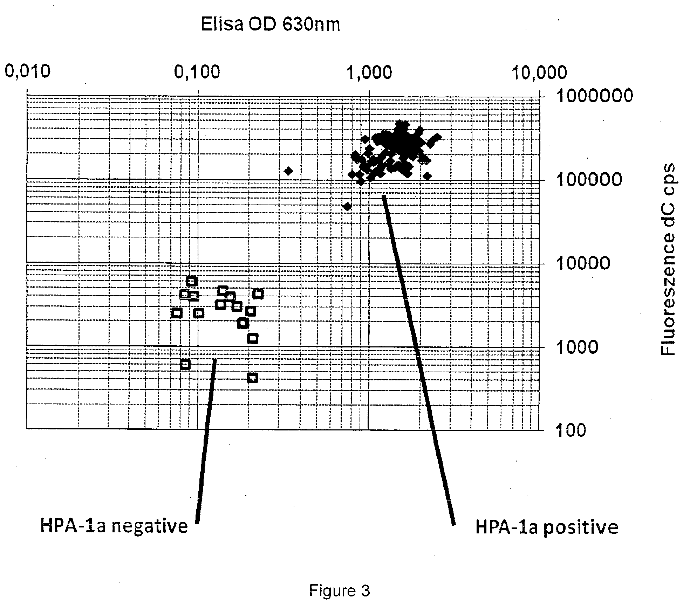

For comparison the blood samples were analyzed with the HPA-1a typing assay of DiaMed, Cressier FR, Switzerland. The result shown in Figure 3 is obtained by plotting on the x-axis the results with the DiaMed HPA-1a typing assay as OD630nm and on the y-axis the fluorescence results obtained with the evanescence biosensor. More than 200 blood samples with known phenotype were tested and no discrepancy was observed.

### Example 2 (Reference Example):

### HPA-5b typing assay

A simple HPA-5b typing assay with the evanescence biosensor system according to the present invention is performed by reacting an anti-gplalla antibody coated surface (anti-gpIaIIa monoclonal antibody AK7), one part of ETDA anti-coagulated blood and two parts of a detection mixture containing an anti HPA-5b specific antibody conjugated to Allo-phycocyanin (APC). Methods and buffers were the same as in example 1 for HPA-1a typing.

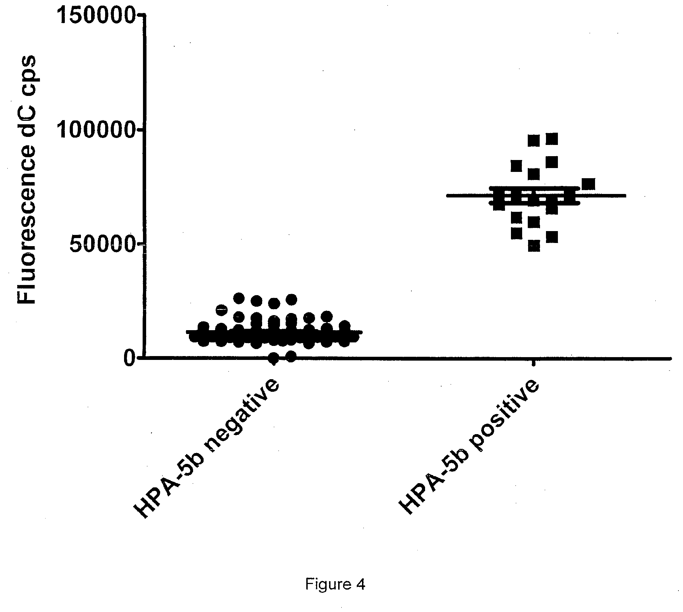

More than 100 blood samples with known phenotypes for HPA 5a and 5b were tested and the results are shown in Figure 4. There is a clear separation of HPA-5aa negative platelets and HPA-5ab positive platelets. No discrepancy between known phenotype and result obtained with the evanescence biosensor assay was observed. Tests with HPA-5bb, and HPA5aa platelets confirm the specificity of the assay.

### Example 3 (Reference Example):

### MAIPA assay using evanescence detection

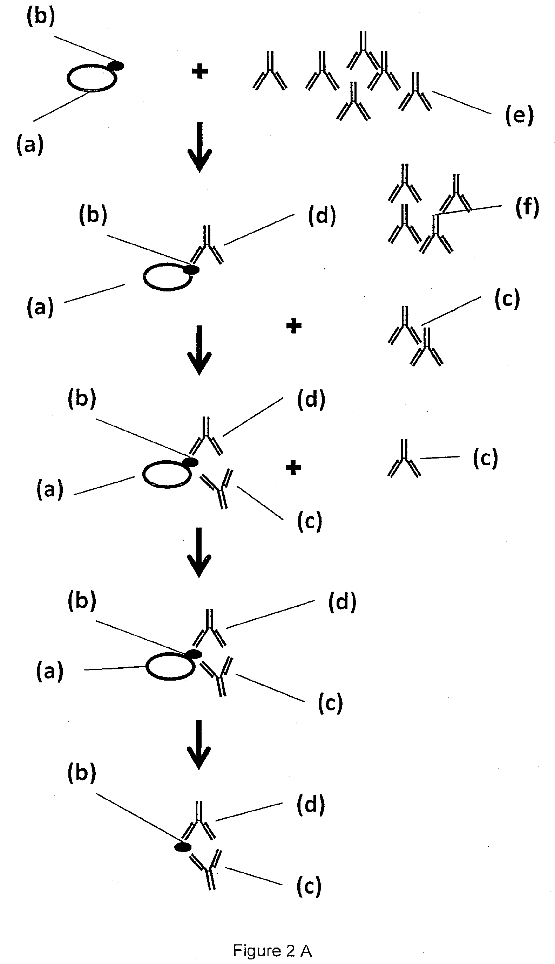

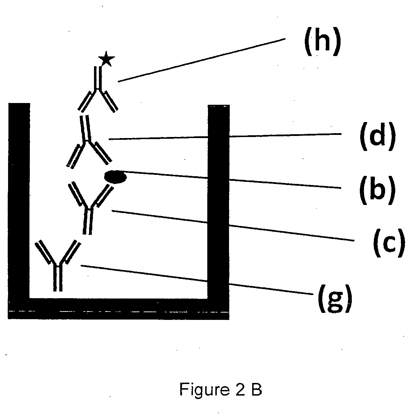

A MAIPA assay has a cellular assay part sensitizing platelets with anti-glycoprotein antibodies and the antibody from human plasma or serum to be detected as sketched in Figure 2A, and a detection part shown in Figure 2B.

Platelet (a) with the platelet glycoprotein (b) are reacted with a monoclonal antibody (c) directed against the platelet glycoprotein under examination. Typically, four reactions for four different glycoproteins are done with monoclonal antibodies against gpIIbIIIa, gplalla, gpIbIX and HLA/beta-2-microglobulin. After the complex is formed by interaction of (b) and (c), the human plasma or serum sample to be analyzed (e) is added and binding of the human anti-HPA antibody (d) occurs, leaving behind the serum with a reduced or depleted anti-platelet antibody (f). Excess human plasma is washed off manually. Using a detergent containing lysis buffer, the tri-molecular complex made of glycoprotein specific antibody (c), platelet glycoproteins (b) and human immunoglobulin (d) is solubilized.

The detection of the tri-molecular complex (b), (c), (d) is then done in a one-step evanescence assay according to the present invention. An anti-mouse IgG antibody (g) is immobilized at 10 microgram per milliliter as described in Example 1 on the bottom of the biosensor well, excess reagent is washed off followed by a blocking step. To improve stability of the coated antibody, the wells are then washed twice with PBS followed by two washes with a 2% sucrose solution The biosensor well is then air dried and kept dry till use. To perform a test, antibody coated platelets are solubilized with a buffer containing 2% BSA, 0.25% PVP, 10mM Hepes, 0.9% NaCl, 0.5% Triton, 0.09% NaN₃. The lysate is then supplemented with 0.04% Brilliant Black BN and an anti-human IgG APC conjugate (Jackson) at 5 microgram per milliliter and measured for 10 min in the fluorescence biosensor instrument.

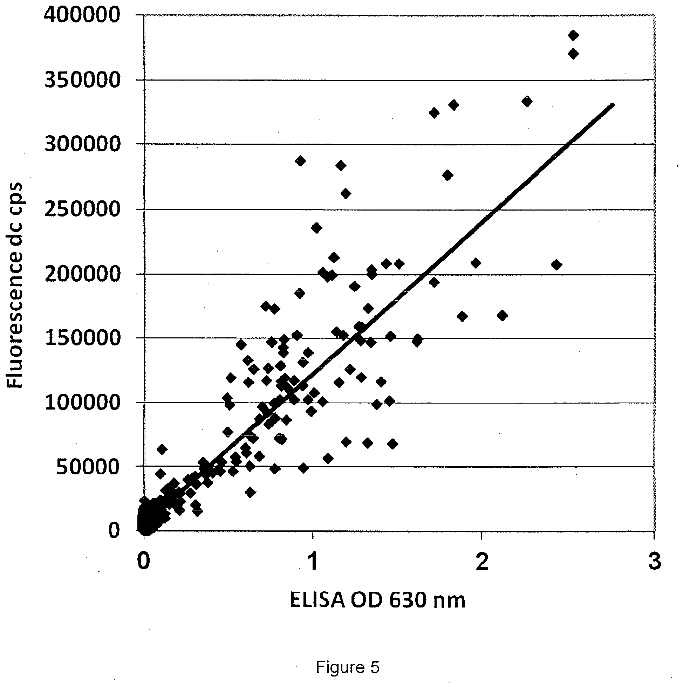

Over 100 serum samples were measured with well over 400 individual measurement points done by a classical MAIPA with ELISA detection or evanescence biosensor detection according to the present invention. The results are shown in Figure 5 with plotting on the x-axis the results with established MAIPA assays and on the y-axis the fluorescence results obtained with the evanescence biosensor. The correlation between the two methods is 0.84 and no discrepancy between the detection methods is observed.

### Example 4 (Reference Example):

### MAIPA Assay using evanescence detection - One tube MAIPA

The MAIPA assay routinely uses four individual tubes where each tube is supplemented with a platelet glycoprotein specific monoclonal antibody, *i.e.* tube one uses an anti-gpIIbIIIa antibody, tube two uses an anti-gpIaIIa antibody, tube three uses an anti-gpIbIX antibody and the fourth tube an anti HLA antibody. The end user must perform an antibody screening test with four individual assays. To reduce the complexity, the assay was performed as a single tube antibody screening assay and to a pool of platelets a pool of the four glycoprotein specific monoclonal antibodies was added and the binding analyzed with the evanescence biosensor assay according to the present invention. Methods and buffers were otherwise identical to the ones given in Example 3.

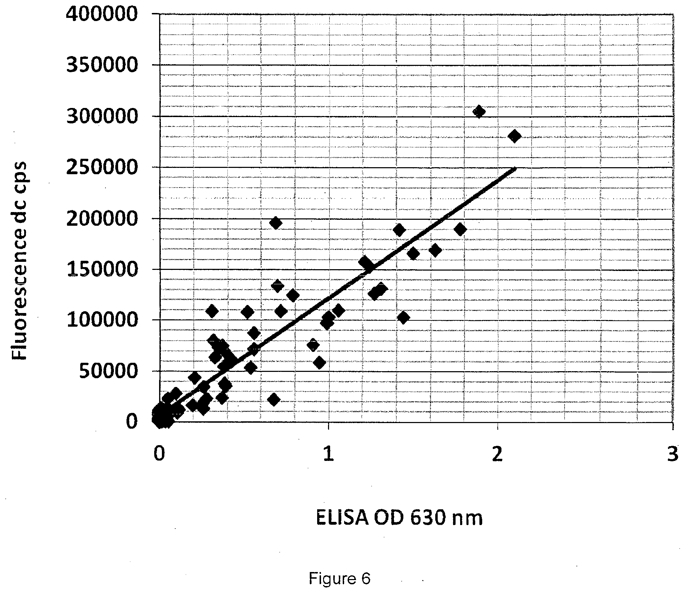

100 serum samples were measured by the classical MAIPA with ELISA detection or evanescence biosensor detection according to the present invention. The results are shown in Figure 6 with plotting on the x-axis the results with established MAIPA assays and on the y-axis the fluorescence results obtained with the evanescence biosensor. The correlation between the two methods is 0.84 and no discrepancy between the two detection methods is observed. In comparison to the classical MAIPA with four individual tube reactions and ELISA detection, the new method uses one tube only with all four monoclonal antibodies and evanescence detection of the resulting tri-molecular complexes. The advantage of the new methods is a highly reduced workload in the cellular part and in the detection part of the assay with identical sensitivity. This now enables the modified single tube MAIPA with evanescence biosensor detection to be done in one third of the time needed for a classical MAIPA with one fourth of the manipulation steps needed and a much reduced complexity for the handling procedures. The new format is now suitable as an antibody screening test for anti-platelet antibodies.

### Example 5:

### HPA-1a antibody assay

A simple HPA-1a antibody assay with the evanescence biosensor system according to the present invention is performed by reacting a platelet coated biosensor surface with an anti HPA-1a monoclonal antibody and after washing away excess unbound anti HPA-1a antibody, detection of the fraction of bound anti HPA-1a is performed with a secondary anti-human IgG APC labeled antibody.

Coating of the biosensor with platelets is done by washing platelets 3 times in PBS and then coating washed platelets in PBS at about 150.000 per microliter in PBS for 1 h at room temperature. This is followed by three PBS washes and a hypotonic lysis in 10mM phosphate buffer with 10mM NaCl for 1 h at RT. Then there are three washes with PBS and a partial protease digestion with Papain for 1h at 37°C (ID-Papain, DiaMed, Cressier, Switzerland) followed by three washes with PBS and then a blocking step with 3% BSA in PBS for 1 h. Wells are washed three times with PBS, two times with 1% sucrose and then air dried after which they are ready-to-use.

The binding assay tests various amounts of anti HPA-1a monoclonal antibody diluted in fetal calf serum, FCS, containing 10mM EDTA for 10 min on the biosensor surface, washing the wells three times with PBS and detecting bound anti HPA-1a IgG with anti-human IgG APC (Jackson) IgG at 5 microgram per ml in FCS supplemented with 10mM EDTA. Measurements are as in Example 1 with the evanescence biosensor for 10 min.

The results shown in Fig. 7 are obtained by plotting on the x-axis the concentration of anti-HPA1a antibody and on the y-axis the fluorescence results in fluorescence dc cps as obtained with the evanescence biosensor. Specificity with respect to platelet genotype 1a and 1b is correct. One observes at low concentrations a linear dose response curve which at concentrations above 1 microgram per milliliter of antibody enters a plateau.

Immobilization of platelets to the sensor surface can be effected with many different immobilization technologies. Possibilities are for example a direct coating by physical absorption, other possibilities are to coat an anti-platelet surface proteins antibody such as for example anti-gpllbllla clone RFGP56 or to biotinylate whole intact platelets leading to a biotinylation of platelet surface proteins and subsequently binding to an avidin biosensor surface. Dextran coating and covalent coupling or Poly-L-Lysine coatings followed by electrostatics and/or covalent couplings are just examples of technical possibilities in this respect. Other anti-platelet antibodies such as anti-HPA-5b or anti-platelet auto-antibodies can be detected using the same method.

### Example 6:

### HPA-1a antibody assay with human plasma samples

The performance of the HPA-1a antibody assay described in Example 5 was further evaluated with a small number of human sera. Assay production and test method and buffers were as described in the Example 5. The evanescence biosensor system is performed by reacting a platelet coated biosensor surface with the anti HPA-1a monoclonal antibody and after washing away excess unbound anti HPA-1a antibody detection of the fraction of bound antiHPA-1a with a secondary anti-human IgG APC labeled antibody.

The binding assay tested 1% dilutions of six different human plasmas for their ability to bind with platelet coated biosensors coated with HPA1a positive or HPA1a negative platelets. The human plasma samples were four negative human plasma and a positive and a negative control from the Lyon EFS platelet reference laboratory, Lyon France with a MAIPA OD of 0.7 for the positive plasma and < 0.1 OD for the negative plasma. This OD of 0.1 is the cut-off value for MAIP positivity or 7 times stronger signal than the cut-off value.

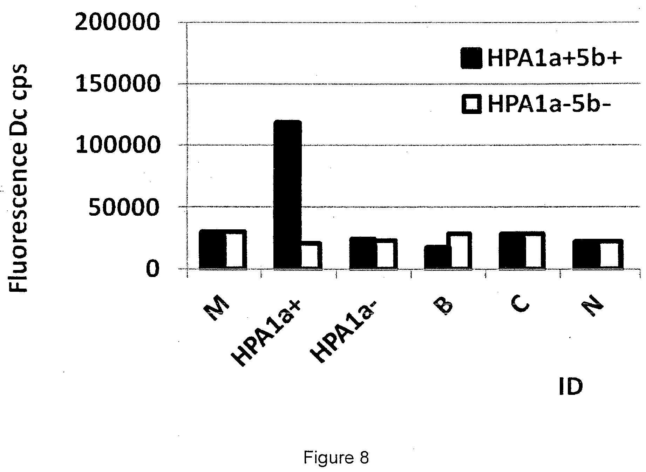

Figure 8 shows the anti HPA-1a positive serum to react strongly with HPA-1a positive platelets and only with a background reaction to HPA-1a negative platelets. Signal-to-noise ratio is similar to the MAIPA results. The HPA1a negative control sera as well as the other negative sera did not give any reaction above background levels.

### Example 7:

### One-step antibody assay for HPA-1a antibody

To further improve the user friendliness in performing a platelet antibody assay platelets biosensors were prepared as described above by coating and drying platelets in the wells.

The assay was then done by diluting the human plasma labeled M from Example 6 by taking one part plasma in 200 parts of FCS supplemented with 10mM EDTA. Two samples were prepared where the first sample used plasma M and the second sample was spiked with an anti-human HPA-1a antibody. Anti-human IgG APC (Jackson) was added to a final concentration of 20 micrograms per milliliter and the mixture was analyzed with a platelet coated biosensor. Three different coatings were used for this assay, HPA1a positive platelets, HPA 1a negative platelets and empty uncoated wells as a negative control for biosensor and instrument background. The mixtures were pipetted into the wells and measured as outlined in the above examples for 10 min in a evanescence biosensor instrument and results were recorded.

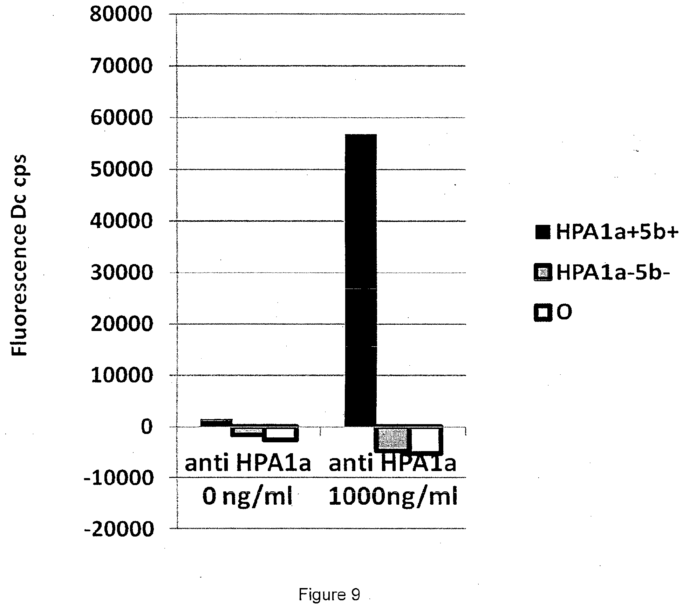

Figure 9 shows a signal only for the HPA1a+ platelets coated biosensor using spiked plasma M whereas the unspiked plasma M gave a negative result. HPA1a negative platelet biosensor and the biosensor alone resulted only in background signal. This confirms the sensitivity and specificity of the evanescence biosensor tests according to the present invention in a one-step antibody assay. It has been demonstrated through the example of HPA-1a alloantigen typing that the evanescence method is suitable for all platelet alloantigen typing and antibody tests.

### Example 8 (Reference Example)

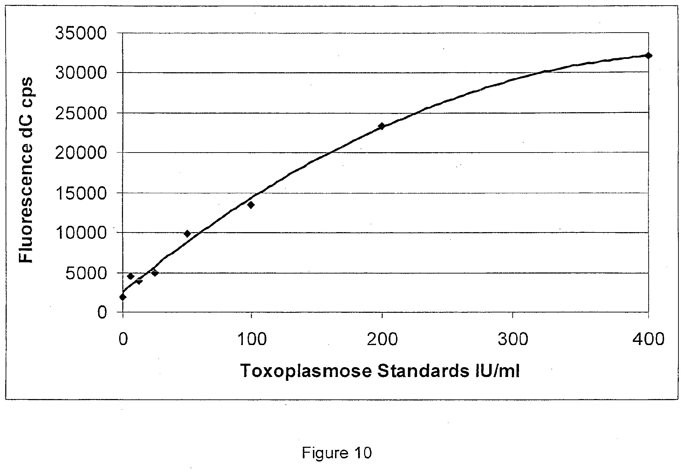

A one-step double antigen antibody assay for toxoplasmose is done by growing toxoplasma in cell culture and purifying the antigen from the culture by repeated sonication and centrifugation steps (DiaMed Eurogen, Turnhout, Belgium). The antigen is in the form of particular matter and used as it is. One aliquot is used to coat the biosensor chip at 10 microgram per milliliter. A second aliquot is labeled with APC as described. The test is done by taking two parts of anti-toxoplasmose IgG standard and mixing with one part of a threefold concentrated detection mix with Tris-buffered 1 % casein, 3% BSA and 0.9% Tween 20 and 10 microgram per milliliter toxoplasmose antigen-APC. This mixture is transferred to a toxoplasmose antigen-coated well and measured. Results are shown in Fig. 10.

## Claims

1. A method for the detection of human antibodies directed against human platelet alloantigens (HPAs), in a sample, comprising the steps:
(a) providing at least one surface having at least human platelets or platelet membrane fractions bound thereto, wherein the HPAs of interest that are present on said platelets or platelet membrane fractions are known;
(b) contacting said surface with said sample, wherein human antibodies directed against the HPAs that are present on said platelets or platelet membrane fractions, contained in said sample, are immobilized on said surface by specifically binding to the platelets or platelet membrane fractions of step (a);
(c) contacting said surface with a fluorescently labeled detection agent, wherein said detection agent specifically binds to human antibodies directed against the HPAs that are present on said platelets or platelet membrane fractions, and wherein said detection agent, respective human antibodies contained in said sample, and the human platelets or platelet membrane fractions of step (a) form a surface-bound complex;
(d) exciting said surface-bound complex with an evanescence field of a light source;
(e) measuring the fluorescence emitted from said surface-bound complex; and
(f) detecting human antibodies against the HPAs that are present on said platelets or platelet membrane fractions based on the emitted fluorescence measured in step (e),
wherein the platelets or platelet membrane fractions are platelets or platelet fragments that comprise native glycoproteins.

2. The method of claim 1, wherein the HPAs are HPA-1a and/or HPA-5b.

3. The method of claim 1 or claim 2, wherein the fluorescently labeled detection agent of step (c) is selected from the group consisting of
(i) fluorescently labeled antibodies directed against human antibodies, and
(ii) fluorescently labeled platelets or platelet membrane fractions, wherein said platelets or platelet membrane fractions carry the same HPAs as the platelets or platelet membrane fractions of step (a).

4. A method for the concurrent detection of (i) at least one human platelet alloantigen (HPA) on human platelets contained in a sample, and (ii) human antibodies directed against human platelet alloantigens (HPAs), in a sample, comprising the steps of:
(α) performing a method for the detection of at least one human platelet alloantigen (HPA) on human platelets contained in a sample, which is anti-coagulated whole blood in a concentration of more than 25%, comprising the steps:
(a) providing at least one surface having at least an antibody bound thereto, wherein said antibody is directed against the glycoprotein carrying the at least one HPA to be detected;
(b) lysing the platelets contained in the sample and solubilizing the membrane proteins of said platelets;
(c) contacting said surface with the lysed and solubilized sample of step (b), wherein respective glycoproteins contained in said sample are immobilized on said surface by specifically binding to the antibody of step (a);
(d) contacting said surface with a fluorescently labeled antibody, wherein said antibody is directed against the at least one HPA to be detected, and wherein said antibody, respective glycoproteins contained in said sample, and the antibody of step (a) form a surface-bound complex;
(e) exciting said surface-bound complex with an evanescence field of a light source;
(f) measuring the fluorescence emitted from said surface-bound complex; and
(g) detecting the at least one HPA based on the emitted fluorescence measured in step (f); and
(β) performing the method of any one of claims 1 to 3.

## Patentansprüche

1. Verfahren für den Nachweis von humanen Antikörpern, welche gegen humane Thrombozyten-Alloantigene (HPAs) gerichtet sind, in einer Probe, umfassend die Schritte:
(a) Bereitstellen mindestens einer Oberfläche, welche mindestens humane Thrombozyten oder Thrombozytenmembran-Fraktionen daran gebunden aufweist, wobei die HPAs von Interesse, welche auf dem Thrombozyten oder Thrombozytenmembran-Fraktionen vorliegen, bekannt sind,
(b) Inkontaktbringen der Oberfläche mit der Probe, wobei humane Antikörper, welche gegen die HPAs gerichtet sind, die auf den Thrombozyten oder Thrombozytenmembran-Fraktionen vorliegen, die in der Probe enthalten sind, auf der Oberfläche durch spezifische Bindung an die Thrombozyten oder Thrombozytenmembran-Fraktionen aus Schritt (a) immobilisiert werden,
(c) Inkontaktbringen der Oberfläche mit einem fluoreszenz-markierten Nachweismittel, wobei das Nachweismittel spezifisch an humane Antikörper bindet, welche gegen die HPAs gerichtet sind, die auf den Thrombozyten oder Thrombozytenmembran-Fraktionen vorliegen, und wobei das Nachweismittel, entsprechende in der Probe enthaltene humane Antikörper, und die humanen Thrombozyten oder Thrombozytenmembran-Fraktionen aus Schritt (a) einen Oberflächen-gebundenen Komplex bilden,
(d) Anregen des Oberflächen-gebundenen Komplexes mit einem Evaneszensfeld einer Lichtquelle,
(e) Messen der von dem Oberflächen-gebundenen Komplex emittierten Fluoreszenz und
(f) Nachweisen von humanen Antikörpern gegen die HPAs, welche auf den Thrombozyten oder Thrombozytenmembran-Fraktionen vorliegen, basierend auf der in Schritt (e) gemessenen emittierten Fluoreszenz,
wobei die Thrombozyten oder Thrombozytenmembran-Fraktionen Thrombozyten oder Thrombozytenfragmente sind, welche native Glykoproteine umfassen.

2. Verfahren nach Anspruch 1, wobei die HPAs HPA-1a und/oder HPA-5b sind.

3. Verfahren nach Anspruch 1 oder Anspruch 2, wobei das fluoreszenz-markierte Nachweismittel von Schritt (c) ausgewählt ist aus der Gruppe, bestehend aus
(i) fluoreszenz-markierten Antikörpern, die gegen humane Antikörper gerichtet sind und
(ii) fluoreszenz-markierten Thrombozyten oder Thrombozytenmembran-Fraktionen, wobei die Thrombozyten oder Thrombozytenmembran-Fraktionen die gleichen HPAs tragen, wie die Thrombozyten oder Thrombozytenmembran-Fraktionen aus Schritt (a).

4. Verfahren für den gleichzeitigen Nachweis von (i) mindestens einem humanen Thrombozyten-Alloantigen (HPA) auf humanen Thrombozyten, enthalten in einer Probe, und (ii) humanen Antikörpern, gerichtet gegen humane Thrombozyten-Alloantigene (HPAs) in einer Probe, umfassend die Schritte:
(α) Durchführen eines Verfahrens für den Nachweis von mindestens einem humanen Thrombozyten-Alloantigen (HPA) auf humanen Thrombozyten, enthalten in einer Probe, welche antikoaguliertes Vollblut in einer Konzentration von mehr als 25 % ist, umfassend die Schritte:
(a) Bereitstellen mindestens einer Oberfläche, die mindestens einen daran gebundenen Antikörper aufweist, wobei der Antikörper gegen das Glykoprotein gerichtet ist, welches das mindestens eine nachzuweisende HPA trägt,
(b) Lysieren der in der Probe enthaltenen Thrombozyten und löslich machen der Membranproteine der Thrombozyten,
(c) Inkontaktbringen der Oberfläche mit der lysierten und löslich gemachten Probe aus Schritt (b), wobei entsprechende in der Probe enthaltene Glykoproteine auf der Oberfläche durch spezifische Bindung an den Antikörper aus Schritt (a) immobilisiert werden,
(d) Inkontaktbringen der Oberfläche mit einem fluoreszenz-markierten Antikörper, wobei der Antikörper gegen das mindestens eine nachzuweisende HPA gerichtet ist und wobei der Antikörper, entsprechende in der Probe enthaltene Glykoproteine und der Antikörper aus Schritt (a) ein Oberflächen-gebundenen Komplex bilden,
(e) Anregen des Oberflächen-gebundenen Komplexes mit einem Evaneszenzfeld einer Lichtquelle,
(f) Messen der von dem Oberflächen-gebundenen Komplex emmitierten Fluoreszenz und
(g) Nachweisen des mindestens einen HPAs, basierend auf der in Schritt (f) gemessenen emittierten Fluoreszenz, und
(β) Durchführen des Verfahrens nach einem der Ansprüche 1 bis 3.

## Revendications

1. Procédé pour la détection d'anticorps humains dirigés contre des allo-antigènes plaquettaires humains (HPA), dans un échantillon, comprenant les étapes suivantes :
(a) fourniture d'au moins une surface ayant au moins des plaquettes humaines ou des fractions de membranes plaquettaires qui leur sont liées, les HPA d'intérêt qui sont présents sur lesdites plaquettes ou fractions de membranes plaquettaires étant connus ;
(b) mise en contact de ladite surface avec ledit échantillon, les anticorps humains dirigés contre les HPA qui sont présents sur lesdites plaquettes ou fractions de membranes plaquettaires, contenues dans ledit échantillon, étant immobilisés sur ladite surface par liaison spécifique aux plaquettes ou fractions de membranes plaquettaires de l'étape (a) ;
(c) mise en contact de ladite surface avec un agent de détection marqué par fluorescence, ledit agent de détection se liant spécifiquement aux anticorps humains dirigés contre les HPA qui sont présents sur lesdites plaquettes ou fractions de membranes plaquettaires, et ledit agent de détection, les anticorps humains respectifs contenus dans ledit échantillon, et les plaquettes ou fractions de membranes plaquettaires humaines de l'étape (a) formant un complexe lié à la surface ;
(d) excitation dudit complexe lié à la surface avec un champ d'évanescence d'une source de lumière ;
(e) mesure de la fluorescence émise depuis ledit complexe lié à la surface ; et
(f) détection des anticorps humains dirigés contre les HPA qui sont présents sur lesdites plaquettes ou fractions de membranes plaquettaires sur la base de la fluorescence émise mesurée dans l'étape (e),
dans lequel les plaquettes ou fractions de membranes plaquettaires sont des plaquettes ou fractions de membranes plaquettaires qui comprennent des glycoprotéines natives.

2. Procédé selon la revendication 1, dans lequel les HPA sont le HPA-1a et/ou le HPA-5b.

3. Procédé selon la revendication 1 ou la revendication 2, dans lequel l'agent de détection marqué par fluorescence de l'étape (c) est sélectionné dans le groupe constitué par :
(i) des anticorps marqués par la fluorescence dirigés contre des anticorps humains, et
(ii) des plaquettes ou fractions de membranes plaquettaires, lesdites plaquettes ou fractions de membranes plaquettaires portant les mêmes HPA que les plaquettes ou fractions de membranes plaquettaires de l'étape (a).

4. Procédé pour la détection simultanée (i) d'au moins un allo-antigène plaquettaire humain (HPA) sur des plaquettes humaines contenues dans un échantillon, et (ii) d'anticorps humains dirigés contre les allo-antigènes plaquettaires humains (HPA), dans un échantillon, comprenant les étapes suivantes :
(α) réalisation d'un procédé pour la détection d'au moins un allo-antigène plaquettaire humain (HPA) sur des plaquettes humaines contenues dans un échantillon, qui est du sang total anticoagulé en une concentration supérieure à 25%, comprenant les étapes suivantes :
(a) fourniture d'au moins une surface ayant au moins un anticorps lui étant lié, ledit anticorps étant dirigé contre la glycoprotéine portant l'au moins un HPA à détecter ;
(b) lyse des plaquettes contenues dans l'échantillon et solubilisation des protéines membranaires desdites plaquettes ;
(c) mise en contact de ladite surface avec l'échantillon lysé et solubilisé de l'étape (b), les glycoprotéines respectives contenues dans ledit échantillon étant immobilisées sur ladite surface par liaison spécifique à l'anticorps de l'étape (a) ;
(d) mise en contact de ladite surface avec un anticorps marqué par fluorescence, ledit anticorps étant dirigé contre l'au moins un HPA à détecter, et ledit anticorps, les glycoprotéines respectives contenues dans ledit échantillon, et l'anticorps de l'étape (a) formant un complexe lié à la surface ;
(e) excitation dudit complexe lié à la surface par un champ d'évanescence d'une source de lumière ;
(f) mesure de la fluorescence émise depuis ledit complexe lié à la surface ; et
(g) détection de l'au moins un HPA sur la base de la fluorescence émise mesurée dans l'étape (f) ; et
(β) réalisation du procédé selon l'une quelconque des revendications 1 à 3.
